Europäisches Patentamt

European Patent Office

Office européen des brevets

(11) Publication number: **0 216 177 B1**

# EUROPEAN PATENT SPECIFICATION

(45) Date of publication of patent specification: **13.03.91**

(51) Int. Cl.⁵: **G01N 33/53**, G01N 33/543, G01N 33/557, //G01N21/64

(21) Application number: **86111834.7**

(22) Date of filing: **27.08.86**

(54) **Enzymatic immunoassay.**

(30) Priority: **30.08.85 JP 191125/85**

(43) Date of publication of application:
**01.04.87 Bulletin 87/14**

(45) Publication of the grant of the patent:
**13.03.91 Bulletin 91/11**

(84) Designated Contracting States:
**BE DE FR GB IT NL**

(56) References cited:
**EP-A- 0 106 324**
**DE-A- 2 749 956**
**DE-A- 2 811 228**

(73) Proprietor: **TOSOH CORPORATION**
**4560, Oaza-Tonda Shinnanyo-shi**
**Yamaguchi 746(JP)**

(72) Inventor: **Higo, Yuji**
**3-24, Tenjin-cho Showa-ku**
**Nagoya-shi(JP)**
Inventor: **Kamada, Satoru**
**D-3-102, 4-7, Tsukimino**
**Yamato-shi Kanagawa-ken(JP)**

(74) Representative: **Patentanwälte Grünecker,**
**Kinkeldey, Stockmair & Partner**
**Maximilianstrasse 58**
**W-8000 München 22(DE)**

## Description

The present invention relates to an enzymatic immunoassay by which a minute amount of biological substances can be detected and/or estimated.

As one of known immunological methods to detect and/or estimate a minute amount of biological substances, the enzymatic immunoassay is recently recognized as noteworthy and actively developed through investigations, in which an enzyme is used as a label on an antigen-antibody reaction complex. This method is an alternative to the hitherto used radio-immunoassay which is carried out using radioisotopes and requires much cares in handling.

The enzymatic immunoassay may be realized in some different manners, as taught for example in "Clinical Chemistry", Vol. 22, No. 8, l243-l255-(l976). However, in general, the procedure goes as follows. At first an antigen or an antibody is affixed to the surface of the inner wall of a cell as reaction chamber or on the surface of beads placed in the cell, the antigen or antibody is brought into an antigen-antibody reaction with a conjugate which is combined with an enzyme as label to form a complex, then a substrate is introduced in the cell which, by the activity of the enzyme, gives rise to an optically detectable change (such as in fluorescent intensity, absorbance, and emission intensity), the change is measured by an optical means such as a fluoro-photometer, and an absorbance photometer, and from the result of measurement the amount of the complex containing enzyme, or hence the amount of the antigen or antibody.

In the enzymatic immunoassay which is concerned with the determination of an amount as minute as $10^{-13}$ of biological substances in general, a relatively large error is involved in the estimation and therefore a high performance of the instrument for measurements as well as removal of error sources are ardently required.

With these points in mind, the present inventors investigated the conventional processes in general of enzymatic immunoassay and found the problems described below.

The processes in the enzymatic immunoassay are generally carried out as follows: at first, an antigen-antibody-enzyme complex is formed affixed to the inner wall of the cup as the reaction chamber, on the surface of beads placed in the cup, or on an insoluble carrier, and then the complex is brought into contact with a substrate solution to start the enzyme reaction. The enzymatic immunoassay is conducted by measuring the fluorescence intensity of the substrate after a certain period of time, either stopping the enzyme reaction with a stopping solution or not. However, in these processes, the time from the initiation of enzyme reaction to the measurement should be strictly controlled, and so-called zero point correction is needed for the fluorescence intensity of the substrate because the intensity at the starting point is not always zero but shows some dispersion. Further, in the characteristic curve expressing the increase of the fluorescence intensity at the substrate, the rate of increase in the intensity becomes gradually smaller from the initiation of the enzyme reaction, and in addition the characteristic curve varies depending on the difference in the amount of enzyme. The correlation between two curves is not linear and hence errors due to difference in the measuring time are different in magnitude and depend individually on the characteristic curves.

Reference EP-A-l06 324 discloses a method for identifying and quantifying allergen specific IgE levels by conjugating the IgE with allergens adhering to an insoluble support, conjugating the IgE with an enzyme labelled anti-IgE antibody, contacting the enzyme label with a solution of substrate which will yield a fluorescent product in the presence of the enzyme and the level of fluorescence in the solution.

Reference DE-A-28 II 228 discloses the estimation of the maximal rate of change of the light intensity in the course of the reaction.

DE-A-27 49 956 discloses kinetic measurements of an immunological reaction with the aid of carrier beads.

Summary of the Invention:

To solve the above-mentioned problems, an object of the present invention is to improve the accuracy and precision of measurements in the enzymatic immunoassay.

Another object of the present invention is to realize a high precision measurement without any heavier load in controlling the process and procedure.

Brief Explanation of the Drawings:

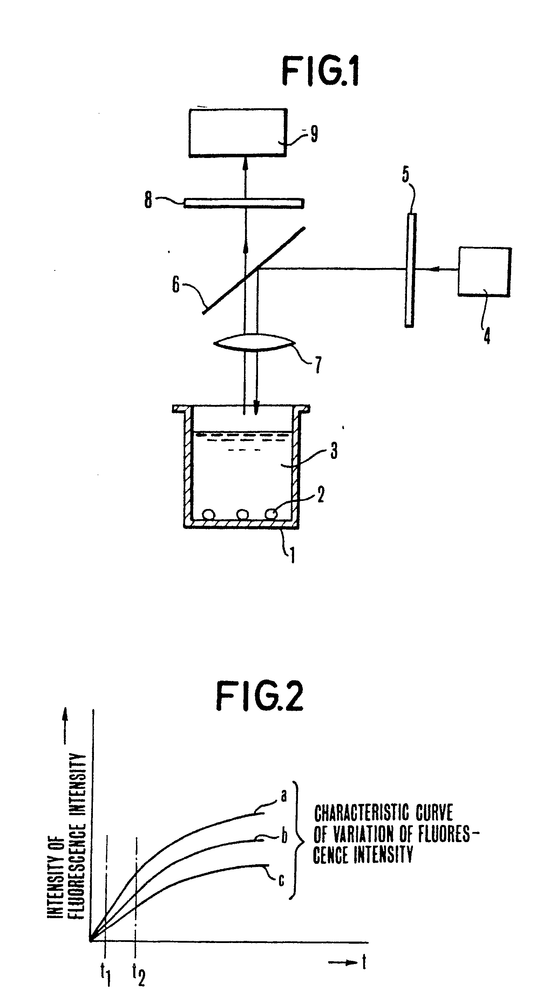

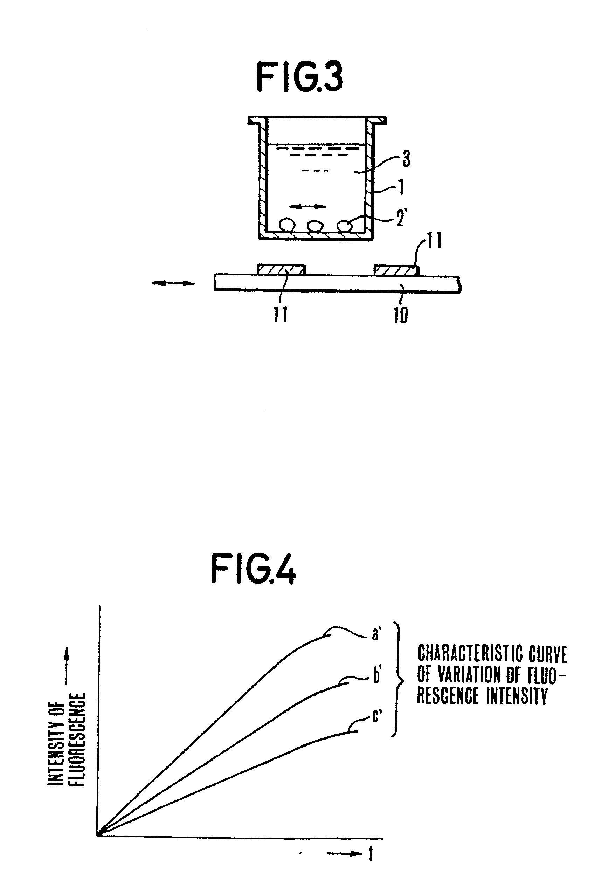

Fig. l is a sketch showing an example of the constitution of an optical system to which the present invention can be applied. Fig. 2 shows the characteristic curves of variation of fluorescence intensity obtained by the the apparatus to which the present invention is applied and Fig 4 is to show the characteristic curves of variation of fluorescence intensity obtained by the apparatus in Fig 3.

Detailed Description of the Invention:

The characteristic features of the present invention which intends to realise the above-mentioned objects are as follows:

Enzymatic immunoassay for estimating the concentration of antigen or antibody comprising bringing an antigen-antibody complex, which is labelled by an enzyme and is present heterogeneously in a solution, as affixed to the surface of insoluble carrier beads, into contact with a substrate where the pH of the solution is so adjusted as to be suitable for the enzyme to be activated and also for measuring the fluorescence of the substrate with an optical system, measuring the time-variation of fluorescent intensity of the substrate produced by the enzyme reaction, and estimating the concentration of the antigen or the antibody from the slope of the substantially straight linear portion, as estiamted from at least two points on a characteristic curve representing variation of the fluorescence intensity, characterized in that the time-variation of the fluorescence intensity of the substrate is measured while the beads are being oscillated at a low frequency in the cell.

The reason why the above-mentioned composition has been adopted is as follows.

The intensity of fluorescence of a substrate as a result of an enzyme reaction depends on the concentration of the substrate and the amount of enzyme (hence the amount of the antigen-antibody-enzyme complex), and directly on the amount of the enzyme when the conoentration of substrate is constant. In a heterogeneous reaction where an antigen-antibody-enzyme complex is formed affixed to the surface of an insoluble carrier such as beads mentioned above and a substrate is brought into contact with the complex, observations in detail of the enzyme reaction as a variation of the fluorescence intensity reveal the substantially linear increase of the fluorescence intensity in the initial period of the enzyme reaction. In this linear region, the slope of the characteristic curve can be significantly estimated with a sufficiently small error, and the slope (or rate of increase) may linearly depend on the amount of enzyme in the solution so far as the concentration of substrate is constant. The present invention which is based on the values thus measured is adopted as useful anywhere.

The system, of which an antigen-antibody-enzyme complex can be present heterogeneously in a solution affixed on the surface of an insoluble carrier, can be an object of this invention. The insoluble carriers are exemplified by the inner wall of a vessel provided as cell, the surface of beads placed in the cell, and textiles and unwoven textiles.

The rate of increase of the fluorescence intensity, that is constant in the initial linear portion, gradually decreases as time goes on, as pointed out before. This is because the complex that is present heterogeneously in the solution undergoes the enzyme reaction which is rate-controlled by the diffusion of the substrate in the solution except in the initial stage of reaction. Therefore, for the purpose of expanding the interval between the two points of measurement to improve the precision in estimating the slope, the insoluble carrier is oscillated at a low frequency to impel the reaction as diffusion-controlled. For the end, the magnetic beads placed in the cell can be oscillated by an oscillating magnetic field. The frequency is selected usually to be l0 - l80/min. preferably 70 - l20/min, and where beads are employed, they are preferably moved in an alternating linear or circular movement throughout the entire part of the cell bottom. Devices for the oscillation of beads may consist of a magnetically permeable cup, magnetic beads composed of magnetic (preferably paramagnetic) powder such as ferrite combined with a resin binder made from polystyrene and EVA, and a magnetic device that generates an oscillating field, for example, by a permanent magnet which is moved in alternating directions. A known process may be followed to affix an antigen or antibody to the surface of beads.

The antigens and antibodies employed in the enzymatic immunoassay of the present invention are not particularly restricted. With regard to the enzyme as label and the substrate, the only necessary condition is that the pH range for activating the enzyme and that suitable for the fluorescence of the substrate overlap each other.

The measurement of fluorescence can be performed with a suitable optical instrument. Thus, the slope of the substantially linear portion can be measured on the observed characteristic curve of the fluorescence intensity. The result is compared with a calibration graph which is prepared beforehand, to obtain the amount of enzyme, hence the amount of the antigen or the antibody.

Detailed Description of a Reference example and the Preferred Embodiment:

The embodiment of the present invention and a reference will be described below with reference to the attached drawings.

Fig. l and 2 refer to the reference example which differ from the present not oscillated invention only in that the beads are not oscillated

In Fig. l, l is a test cup, 2 is beads placed in the cup l (dimension of the beads is not restricted, but preferably equal to or less than half that of the diameter of the cell), and an antigen-antibody-enzyme complex is formed affixed on the surface of beads by a usual process. 3 is a substrate solution, the pH of which for the measurement of fluorescence intensity is selected so as to be matched with that of activating the enzyme.

Measuring the fluorescence intensity is started by means of a fluorescence detection apparatus immediately after the addition of both beads 2 and substrate solution 3 to the test cup I.

The fluorescence detection optical system according to the present invention consists of light source 4, filter for exciting light 5, dichroic mirror 6, focusing lens 7, filter for fluorescent light 8 and light sensor 9. Signals detected at the light sensor 9 are transferred via an appropriate amplifier to a computation circuit (including a microcomputer, but not indicated in the drawing) to calculate a characteristic curve on variation of the fluorescence intensity as a function of time.

In Fig. 2, a, b, and c are examples of the characteristic curve on variation of the fluorescence intensity thus obtained, showing the amounts of the antigen (or antibody) in the samples being in the order of a > b > c. Moreover, in the early stage of the enzyme reaction, there appears a substantially linear portion in the region between tl - t2 on each curve in Fig. 2, which is hardly affected by the diffusion. Thus, if the rate of increase (or the slope) of the fluorescence intensity is determined in the above-mentioned region for each sample and compared with that of the calibration graph, determination of the amounts of the antigen or the antibody can be realized.

In this process the zero point correction is not required for the characteristic curves. further, if the time interval between two measuring points is exactly controlled to estimate the rate of increase (or slope), the time for the initiation procedure of the reaction (for example, adding the substrate solution to the test cup) is not particularly required to be controlled.

This is especially advantageous for the apparatus with which many samples are to be dealt continuously, because designing engineers can have a large freedom at their disposal in deciding the time point of adding a substrate solution to the test cup, and this is invaluable in practice. Fig. 3 and 4 refer to the preferred embodiment of the invention.

In Fig. 3, beads 2' are magnetic bodies and influenced by a magnetic field and a number of permanent magnets II are fixed at appropriate intervals on a bar 10 that moves alternatingly closely beneath the test cup I. In operation, the beads undergo an alternating movement in the test cup I in accordance with the alternatingly moving permanent magnets II. The characteristic curves of thus obtained of the variation of fluorescence intensity as a function of time, indicated as a', b' and c', contain substantially linear portions which are longer than those obtained otherwise. Therefore, the rate of increase (or slope) can be estimated with a better precision than in the reference example in Fig. I, and this is markedly effective for the determination of a minute amount of biological substances.

Reference

Using the apparatus in Fig. I, a complex which was labelled with an enzyme alkaline phosphatase was formed on the surface of a bead, and a substrate solution containing 4-methylumbelliferone (I mmol/l, pH 9.5) was poured in the test cup I and the intensity of fluorescence was measured.

The characteristic curve obtained showing the variation of fluorescence intensity as a function of time contained a substantially linear portion from the addition of the substrate solution to the elapse of 60 sec. The slope of the curve as measured using two arbitrary points in the range was obtained with an error not more than 0.I % in a plurality of runs using the same sample.

Example I:

Following the example in Fig. 3, the same test as in was conducted using I0 beads of I.4 mm in diameter which were oscillated at a frequency of 90/min.

In this case, a substantially linear portion appeared on the characteristic curve of variation of fluorescence intensity as a function of time in the range from the injection of the substrate solution to the elapse of 60 sec. The slope as measured from two points on the curve was obtained with an error not more than 0.I % in a plurality of runs using the same sample.

As has been mentioned above, the process of the present invention permits the determination of a minute amount of a biological substance with high accuracy and precision, and further the requirements to correct the zero point offset and to strictly control the time for the procedure is either lessened or rendered irrelevant, and thus achieves great advantages. In addition, when an apparatus is assembled to treat a number of samples in a continuous manner, the present invention provides a large degree of freedom in the design of apparatus, also giving a great advantage.

Claims

1. Enzymatic immunoassay for estimating the concentration of antigen or antibody comprising bringing an antigen-antibody complex, which is labelled by an enzyme and is present heterogeneously in a solution, as affixed to the surface of insoluble carrier beads, into contact with a substrate where the pH of the solution is so adjusted as to be suitable for the enzyme to be activated and also for measuring the fluorescence of the substrate with an optical system, measuring the time-variation of fluorescent intensity of the substrate produced by the enzyme reaction, and estimating the concentration of the antigen or the antibody from the slope of the substantially straight linear portion, as estimated from at least two points, on a characteristic curve representing variation of the fluorescence intensity, characterized in that the time-variation of the fluorescence intensity of the substrate is measured while the beads are being oscillated at a low frequency in the cell.

2. Enzymatic immunoassay according to claim I characterized in that the carrier beads are magnetic particles.

3. Enzymatic immunoassay according to at least one of the foregoing claims characterized in that the oscillation of beads is affected by a magnetic member arranged outside the cell.

4. Enzymatic immunoassay according to at least one of the foregoing claims characterized in that the frequency of oscilliation from I0 to I80/min.

5. Enzymatic immunoassay according to at least one of the foregoing claims characterized in that the optical system for measuring the fluorescence is a top-to-top type arranged above the upper-opening cell (figure I).

**Revendications**

1. Essais immunologiques enzymatiques pour estimer la concentration d'un antigène ou d'un anticorps comprenant les étapes consistant à amener un complexe antigène-anticorps, qui est marqué par un enzyme et est présent de façon hétérogéne dans une solution, comme étant fixé à la surface de perles de support insoluble, en contact avec un substrat où le pH de la solution est ajusté de façon à être approprié à l'enzyme à activer et aussi pour mesurer la fluorescence du substrat avec un système optique, à mesurer la variation dans le temps de l'intensité fluorescente du substrat produite par la réaction de l'enzyme, et à estimer la concentration de l'antigéne ou de l'anticorps à partir de la pente de la partie linéaire sensiblement rectiligne, telle qu'estimée à partir d'au moins deux points, sur une courbe caractéristique représentant la variation de l'intensité de la fluorescence, caractérisés en ce que la variation dans le temps de l'intensité de la fluorescence du substrat est mesurée alors que les perles sont soumises à des oscillations à une basse fréquence dans la cellule.

2. Essais immunologiques enzymatiques selon la revendication I, caractérisés en ce que les perles du support sont des particules magnétiques.

3. Essais immunologiques enzymatiques selon au moins l'une des revendications précédentes, caractérisés en ce que l'oscillation des perles est influencée par un élément magnétique disposé à l'extérieur de la cellule.

4. Essais immunologiques enzymatiques selon au moins l'une des revendications précédentes, caractérisés en ce que la fréquence d'oscillation est comprise entre I0 et I80/mn.

5. Essais immunolgiques enzymatiques selon au moins l'une des revendications précédentes, caractérisés en ce que le système optique pour mesurer la fluorescence est d'un type sommet contre sommet disposé au-dessus de la cellule débouchant vers le haut (figure I).

**Ansprüche**

1. Enzymimmunoassay zum Abschätzen der Konzentration eines Atigenes oder Antikörpers, umfassend

das In-Kontakt-bringen eines Antigen-Antikörper-Komplexes, der mit einem Enzym markiert ist und in einer Lösung heterogen vorhanden ist, beispielsweise an die Oberfläche unlöslicher Trägerkugeln angeheftet, mit einem Substrat, wobei der pH der Lösung so eingestellt ist, daß er für die Aktivierung des Enzymes und außerdem für die Messung der Fluoreszenz des Substrats mit einem optischen System geeignet ist,

das Messen der zeitabhängigen Veränderung der Fluoreszenzintensität des Substrats, die durch die Enzymreaktion erzeugt wird, und

das Abschätzen der Konzentration des Antigenes oder des Antikörpers aus der Steigung des im wesentlichen gerade linearen Teils, abgeschätzt aus mindestens zwei Punkten einer charakteristischen Kurve, die die Veränderung der Fluoreszenzintensität darstellt, **dadurch gekennzeichnet, daß** die zeitabhängige Veränderung der Fluoreszenzintensität des Substrats gemessen wird, während die Kugeln bei einer niedrigen Frequenz in der Zelle in Schwingung versetzt werden.

2. Enzymimmunoassay nach Anspruch I, **dadurch gekennzeichnet**, daß die Trägerkugeln magnetische Teilchen sind.

3. Enzymimmunoassay nach mindestens einem der voranstehenden Ansprüche, **dadurch gekennzeichnet**, daß die Schwingung der Kugeln durch ein magnetisches Teil, das außerhalb der Zelle angeordnet ist, bewirkt wird.

4. Enzymimmunoassay nach mindestens einem der voranstehenden Ansprüche, **dadurch gekennzeichnet**, daß die Schwingung eine Frequenz von I0 - I80/min hat.

5. Enzymimmunoassay nach mindestens einem der voranstehenden Ansprüche, **dadurch gekennzeichnet**, daß das optische System zum Messen der Fluoreszenz eines vom Top-to-Top-Typus ist, das über der oben sich öffnenden Zelle angeordnet ist (Figur I).

# FIG.1

# FIG.2

INTENSITY OF FLUORESCENCE INTENSITY

a
b
c

CHARACTERISTIC CURVE OF VARIATION OF FLUORESCENCE INTENSITY

$t_1$ $t_2$ $t$

# FIG.3

# FIG.4

CHARACTERISTIC CURVE
OF VARIATION OF FLUO-
RESCENCE INTENSITY